# EUROPÄISCHE PATENTSCHRIFT

(11) **EP 0 839 131 B1**
(45) Veröffentlichungstag und Bekanntmachung des Hinweises auf die Patenterteilung: **22.12.1999**
(21) Anmeldenummer: 96924874.9
(22) Anmeldetag: 04.07.1996
(51) Int. Cl.: C07C 323/65, C07C 317/40, C07C 317/36, C07C 317/18

(54) **AROMATISCHE SULFONYLVERBINDUNGEN, DIE EINE ZUSÄTZLICHE THIOETHER-, SULFOXID- ODER SULFONYLGRUPPE AUFWEISEN**
AROMATIC SULPHONYL COMPOUNDS HAVING AN ADDITIONAL THIOETHER, SULPHOXIDE OR SULPHONYL GROUP
COMPOSES DE SULFONYLE AROMATIQUES PRESENTANT UN GROUPE THIOETHER, OXYDE SULFONIQUE OU SULFONYLE SUPPLEMENTAIRE.

(30) Priorität: 14.07.1995 DE 19525679
(43) Veröffentlichungstag der Anmeldung: 06.05.1998
(73) Patentinhaber: BASF AKTIENGESELLSCHAFT, 67056 Ludwigshafen (DE)
(72) Erfinder: MARSCHNER, Claus, D-67346 Speyer (DE); PATSCH, Manfred, D-67157 Wachenheim (DE)
(86) Internationale Anmeldenummer: EP9602929
(87) Internationale Veröffentlichungsnummer: WO9703955

(56) Entgegenhaltungen:
- EP-A- 0 210 951
- JOURNAL OF HETEROCYCLIC CHEMISTRY, Bd. 23, Nr. 2, März 1986 - April 1986, PROVO, US, Seiten 353-360, XP002017215 J. BRADSHAW, ET AL.: "Proton-ionisable crown compounds. 3. Synthesis and structural studies of macrocyclic polyether ligands containing a 4-pyrone subcyclic unit"

## Beschreibung

Die vorliegende Erfindung betrifft neue Sulfonylverbindungen der Formel I 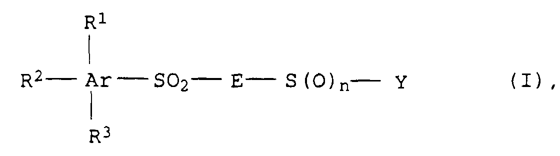 in der
- n: 0, 1 oder 2,
- Y: Vinyl oder einen Rest der Formel C₂H₄Q, worin Q für Hydroxy oder eine unter alkalischen Reaktionsbedingungen abspaltbare Gruppe steht,
- E: C₃-C₆-Alkylen, das durch 1 oder 2 Sauerstoffatome in Ether; funktion unterbrochen sein kann,
- Ar: den Rest von Benzol oder Naphthalin und
- R¹, R² und R³: unabhängig voneinander jeweils Wasserstoff, C₁-C₆-Alkyl, das durch Amino oder C₁-C₄-Alkanoylamino substituiert sein kann, C₁-C₆-Alkoxy, Hydroxy, Halogen, Nitro, Amino, C₁-C₄-Alkanoylamino, Mono- oder Di-C₁-C₆-alkylamino, Hydroxysulfonyl, Carboxyl, Carbamoyl, Mono- oder Di-C₁-C₆-alkylcarbamoyl, Sulfamoyl, Mono- oder Di-C₁-C₆-alkylsulfamoyl, Cyano oder einen Rest der Formel (NH-)ₘ(CH₂-)_{q}SO₂-Y, worin m für 0 oder 1 und q für 0, 2 oder 3 stehen und Y die obengenannte Bedeutung besitzt,
bedeuten.

Aufgabe der vorliegenden Erfindung war es, neue aromatische Sulfonylverbindungen mit einer zusätzlichen Thioether-, Sulfoxid- oder Sulfonylgruppe im Molekül bereitzustellen. Die neuen Verbindungen sollten leicht zugänglich sein und sich vorteilhaft als Zwischenprodukte für Farbstoffe eignen.

Demgemäß wurden die eingangs näher bezeichneten aromatischen Sulfonylverbindungen der Formel I gefunden.

Alle in den hier aufgeführten Formeln auftretenden Alkyl- und Alkylengruppen können sowohl geradkettig als auch verzweigt sein.

Reste R¹, R² und R³ sind z.B. Methyl, Ethyl, Propyl, Isopropyl, Butyl, Isobutyl, sec-Butyl, tert-Butyl, Pentyl, Isopentyl, Neopentyl, tert-Pentyl, Hexyl, 2-Methylpentyl, Aminomethyl, 2-Aminoethyl, 2- oder 3-Aminopropyl, 2- oder 4-Aminobutyl, 5-Aminopentyl, 6-Aminohexyl, Formylaminomethyl, 2-Formylaminoethyl, 2- oder 3-Formylaminopropyl, 2- oder 4-Formylaminobutyl, 5-Formylaminopentyl, 6-Formylaminohexyl, Acetylaminomethyl, 2-Acetylaminoethyl, 2- oder 3-Acetylaminopropyl, 2- oder 4-Acetylaminobutyl, 5-Acetylaminopentyl, 6-Acetylaminohexyl, Methoxy, Ethoxy, Propoxy, Isopropoxy, Butoxy, Isobutoxy, sec-Butoxy, Pentyloxy, Isopentyloxy, Neopentyloxy, tert-Pentyloxy, Hexyloxy, Fluor, Chlor, Brom, Mono- oder Dimethylamino, Mono- oder Diethylamino, Mono- oder Dipropylamino, Mono- oder Diisopropylamino, Mono- oder Dibutylamino, Mono- oder Dipentylamino, Mono- oder Dihexylamino, Mono- oder Dimethylcarbamoyl, Mono- oder Diethylcarbamoyl, Mono- oder Dipropylcarbamoyl, Mono- oder Dibutylcarbamoyl, Mono- oder Dipentylcarbamoyl, Mono- oder Dihexylcarbamoyl, Mono- oder Dimethylsulfamoyl, Mono- oder Diethylsulfamoyl, Mono- oder Dipropylsulfamoyl, Mono- oder Dibutylsulfamoyl, Mono- oder Dipentylsulfamoyl, Mono- oder Dihexylsulfamoyl, Formylamino, Acetylamino, Propionylamino, Butyrylamino oder Isobutyrylamino.

Reste E sind z.B. (CH₂)₃, (CH₂)₄, (CH₂)₅, (CH₂)₆, CH(CH₃)CH₂, CH(CH₃)CH(CH₃), (CH₂)₂O(CH₂)₂, (CH₂)₂O(CH₂)₃, (CH₂)₃O(CH₂)₃ oder (CH₂)₂O(CH₂)₂O(CH₂)₂.

Der Rest Q steht für Hydroxy oder für eine unter alkalischen Reaktionsbedingungen abspaltbare Gruppe. Solche Gruppen sind z.B. Chlor, Brom, C₁-C₄-Alkylsulfonyl, Phenylsulfonyl, OSO₃H, SSO₃H, OP(O)(OH)₂, C₁-C₄-Alkylsulfonyloxy, Phenylsulfonyloxy, C₁-C₄-Alkanoyloxy, C₁-C₄-Dialkylamino oder ein Rest der Formel 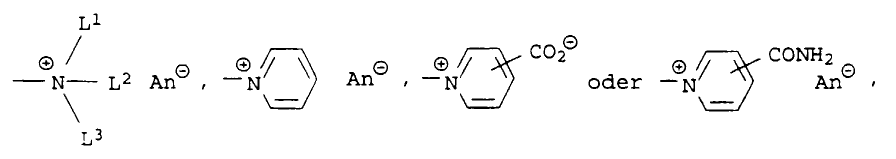 wobei L¹, L² und L³ unabhängig voneinander jeweils die Bedeutung von C₁-C₄-Alkyl oder Benzyl und An⊖ jeweils die Bedeutung eines Äquivalents eines Anions besitzen. Als Anionen können dabei z.B. Fluorid, Chlorid, Bromid, Iodid, Mono-, Di- oder Trichloracetat, Methansulfonat, Benzolsulfonat oder 2- oder 4-Methylbenzol-sulfonat in Betracht kommen.

Bevorzugt sind Sulfonylverbindungen der Formel I, in der n 0 oder 2, insbesondere 2, bedeutet.

Weiterhin bevorzugt sind Sulfonylverbindungen der Formel I, in der E C₃- oder C₄-Alkylen, insbesondere C₃-Alkylen bedeutet.

Weiterhin bevorzugt sind Sulfonylverbindungen der Formel I, in der Ar den Rest von Benzol bedeutet.

Weiterhin bevorzugt sind Sulfonylverbindungen der Formel I, in der R¹ C₁-C₄-Alkyl, das durch Amino oder C₁-C₄-Alkanoylamino substituiert sein kann, Nitro, Amino oder C₁-C₄-Alkanoylamino und R² und R³ unabhängig voneinander jeweils Wasserstoff, C₁-C₄-Alkoxy, Hydroxy, Halogen, Hydroxysulfonyl oder Carboxyl bedeuten.

Besonders bevorzugt sind Sulfonylverbindungen der Formel I, in der R¹ C₁-C₄-Alkyl, das durch Amino oder C₁-C₄-Alkanoylamino substituiert sein kann, Nitro, Amino oder C₁- oder C₂-Alkanoylamino, R² Wasserstoff, C₁-C₄-Alkoxy, Hydroxy, Halogen, Hydroxysulfonyl oder Carboxyl und R³ Wasserstoff oder Hydroxysulfonyl bedeuten.

Von besonderem technischen Interesse sind Sulfonylverbindungen der Formel Ia 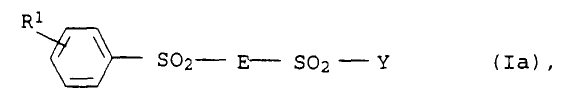 in der
- Y: Vinyl, 2-Sulfatoethyl, 2-Chlorethyl oder 2-Acetyloxyethyl,
- E: C₃- oder C₄-Alkylen und
- R¹: C₁-C₄-Alkyl, das durch Amino oder C₁- oder C₂-Alkanoylamino substituiert sein kann, Nitro, Amino oder C₁- oder C₂-Alkanoylamino
bedeuten.

Die erfindungsgemäßen Sulfonylverbindungen der Formel I können nach an sich bekannten Methoden erhalten werden. Vorteilhaft setzt man dabei z.B. eine aromatische Verbindung der Formel II 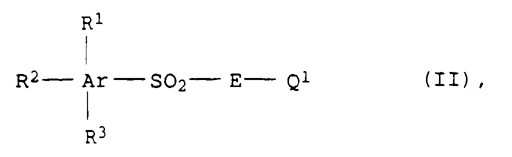 in der E, Ar, R¹, R² und R³ jeweils die obengenannte Bedeutung und Q¹ mit Ausnahme von Hydroxy die Bedeutung des Rests Q besitzen, mit Mercaptoethanol um, wobei die Hydroxyverbindung der Formel Ib 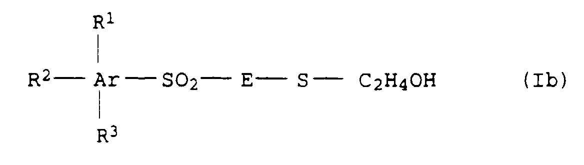 resultiert, in der E, Ar, R¹, R² und R³ jeweils die obengenannte Bedeutung besitzen.

Diese kann dann gegebenenfalls in einer Folgereaktion, z.B. mit Wasserstoffperoxid zum Sulfoxid (n in Formel I = 1) oder Sulfon (n in Formel I = 2) oxidiert und die unter alkalischen Reaktionsbedingungen abspaltbare Gruppe gegebenenfalls anschließend eingeführt werden, beispielsweise durch Veresterung mit Schwefelsäure. Es ist aber auch möglich, die unter alkalischen Reaktionsbedingungen abspaltbare Gruppe zuerst in die Hydroxyverbindung der Formel Ib einzuführen und diese gegebenenfalls anschließend zu oxidieren.

Die neuen Sulfonylverbindungen der Formel I sind wertvolle Zwischenprodukte zur Herstellung von Reaktivfarbstoffen.

Die folgenden Beispiele sollen die Erfindung näher erläutern.

### Beispiel 1

Zu einem Gemisch aus 200 g 4-Acetylaminobenzolsulfinsäure, 500 ml N,N-Dimethylformamid (DMF) und 152 g Kaliumcarbonat wurden bei einer Temperatur von 20 bis 25°C 173 g 1-Brom-3-chlorpropan zugetropft. Anschließend wurde 12 h bei 20 bis 25°C gerührt.

Nach Beendigung der Reaktion (DC-Kontrolle) wurde das Reaktionsgemisch auf 750 g Eis gefällt und 1 h nachgerührt. Der Niederschlag wurde abgesaugt, mit Wasser neutral gewaschen und unter vermindertem Druck bei 50°C getrocknet.

Man isolierte 260 g der Verbindung der Formel 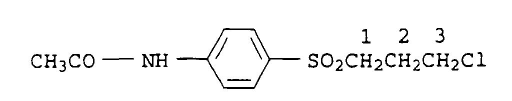
- ¹H-NMR (D₆-DMSO) :: δ = 2,0 (m, 2H, 2-H), 2,1 (s, 3H, CH₃)
3,3 (t, 2H, 3-H)
3,7 (t, 2H, 1-H)
7,8 (brs, 4H, Aromaten-H) ppm.
- ¹³C-NMR (D₆-DMSO) :: δ = 24,1 (CH₃), 26,1 (C-2), 43,0 (C-3)
52,8 (C-1), 118,9 , 128,9 , 132,2
144,2 (Aromaten-C), 169,1 (C=O) ppm.

### Beispiel 2

a) 175 g der in Beispiel 1 beschriebenen Verbindung und 96,6 g Kaliumcarbonat wurden in 950 ml DMF vorgelegt. Bei 60 bis 65°C wurde eine Mischung aus 81,9 g Mercaptoethanol und 220 ml DMF zugetropft. Anschließend wurde das Reaktionsgemisch bis zur vollständigen Umsetzung (DC-Kontrolle) bei dieser Temperatur gerührt.
   Nach dem Abkühlen wurde die Reaktionsmischung mit 20 gew.-%iger wäßriger Kochsalzlösung verdünnt und mehrmals mit Essigester extrahiert. Nach dem Abziehen des Lösungsmittels unter vermindertem Druck isolierte man 175 g der Verbindung der Formel 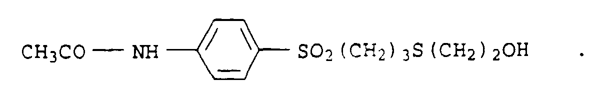
b) Die unter a) beschriebene Verbindung wurde in 500 ml Wasser gelöst. Nach der Zugabe von 0,5 g Wolframsäure wurden bei 20 bis 25°C 342 g 30 gew.-%ige wäßrige Wasserstoffperoxidlösung zugetropft. Das Reaktionsgemisch wurde 2 h auf 70°C erwärmt. Nach beendeter Umsetzung (DC-Kontrolle) wurde der entstandene Niederschlag bei 10°C abgesaugt, mit Wasser gewaschen und unter vermindertem Druck bei ca. 30°C getrocknet.
   Es verblieben 122 g der Verbindung der Formel 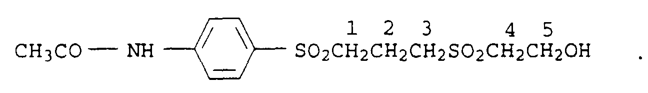
   - ¹H-NMR (D₆-DMSO) :: δ = 2,0 (m, 2H, 2-H)
   2,1 (s, 3H, CH₃)
   3,2 (m, 4H, 4-H, 5-H)
   3,4 (t, 2H, 3-H)
   3,8 (t, 2H, 1-H)
   7,8 (brs, 4H, Aromaten-H)
   11,3 (s, 1H, NH) ppm.
   - ¹³C-NMR (D₆-DMSO) :: δ = 15,8 (C-2)
   24,1 (CH₃)
   51,7 (C-3)
   53,4 (C-1)
   55,0, 55,2 (C-4, C-5)
   118,8 , 128,9 , 132,0 , 144,1 (Aromaten-C)
   169,1 (C=O) ppm.

### Beispiel 3

50 g der in Beispiel 2b beschriebenen Verbindung wurden in 500 g halbkonzentrierter Salzsäure 8 h auf 80 bis 85°C erhitzt. Die Reaktionslösung wurde anschließend unter vermindertem Druck bei 50°C eingeengt. Man isolierte 50 g der Verbindung der Formel 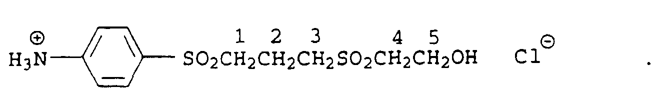
- ¹H-NMR (D₆-DMSO) :: δ = 2,0 (m, 2H, 2-H)
3,3 (m, 6H, 3-H, 4-H, 5-H)
3,8 (t, 2H, 1-H)
6,9 (d, 2H, Aromaten-H)
7,2 (brs, 3H, NH₃⊕)
7,6 (d, 2H, Aromaten-H) ppm.

### Beispiel 4

Zu einem Gemisch aus 186,3 g 3-Nitrobenzolsulfinsäure, 500 ml DMF und 151,4 g Kaliumcarbonat wurden bei einer Temperatur von 20 bis 25°C 172,4 g 1-Brom-3-chlorpropan getropft. Anschließend wurde 16 h bei 20 bis 25°C nachgerührt. Das Reaktionsgemisch wurde mit Wasser verdünnt und mehrmals mit Essigester extrahiert. Nach dem Abziehen des Lösungsmittels unter vermindertem Druck isolierte man 165 g einer Verbindung der Formel 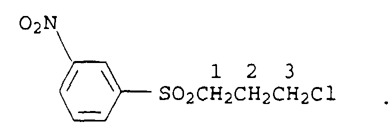
- ¹H-NMR (D₆-DMSO) :: δ = 2,05 (m, 2H, 2-H)
3,6 (t, 2H, 3-H)
3,7 (t, 2H, 1-H)
8,0 (t, 1H, Aromaten-H)
8,4 (d, 1H, Aromaten-H)
8,6 (m, 2H, Aromaten-H) ppm.

### Beispiel 5

a) 162,6 g der in Beispiel 4 beschriebenen Verbindung wurden analog Beispiel 2a mit 94,3 g Kaliumcarbonat und 53,6 g Thioethanol in 650 ml DMF umgesetzt. Man erhielt 145,2 g der Verbindung der Formel 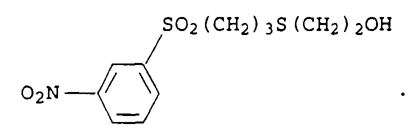
b) Die unter a) beschriebene Verbindung wurde anschließend in 120 ml Wasser mit 256 g 30 gew.-%iger wäßriger Wasserstoffperoxidlösung analog Beispiel 2b oxidiert. Nach dem Trocknen des isolierten Produkts verblieben 124 g der Verbindung der Formel 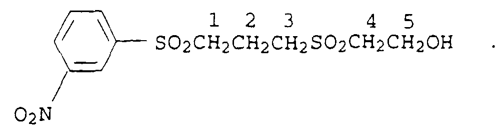
   - ¹H-NMR (D₆-DMSO) :: δ = 2,0 (m, 2H, 2-H)
   3,2 (m, 4H, 4-H, 5-H)
   3,7 (t, 2H, 3-H)
   3,8 (t, 2H, 1-H)
   8,0 (t, 1H, Aromaten-H)
   8,4 (t, 1H, Aromaten-H)
   8,6 (m, 2H, Aromaten-H) ppm.
   - ¹³C-NMR (D₆-DMSO) :: δ = 15,5 (C-2)
   51,7 (C-1)
   52,9 (C-3)
   55,0 , 55,2 (C-4, C-5)
   122,7 , 128,5 , 131,6 , 133,8
   140,3 , 148,1 (Aromaten-C)

### Beispiel 6

33,7 g der in Beispiel 5b) beschriebenen Verbindung wurden in 300 g Methanol und 2 g Propionsäure gelöst. Man gab 5 g Raney-Nickel zu und hydrierte bei 40°C bei einem Wasserstoffdruck von 2 bar. Nach beendeter Wasserstoff-Aufnahme wurde vom Katalysator abfiltriert und das Filtrat vom Lösungsmittel befreit. Es verblieben 30,0 g der Verbindung der Formel 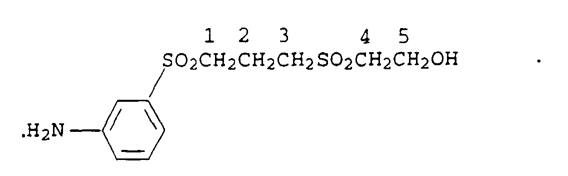
- ¹H-NMR (D₆-DMSO) :: δ = 1,8 (m, 2H, 2-H)
3,1 - 3,7 (m, 8H, 1-, 3-, 4- und 5-H)
4,3 (m, 1H, OH)
5,7 (brs, 2H, NH₂)
6,8 - 7,3 (t, 4H, Aromaten-H)

### Beispiel 7

Man verfuhr analog Beispiel 1, verwendete jedoch anstelle von 1-Brom-3-chlorpropan 188 g 1-Brom-4-chlorbutan. Man erhielt 262 g der Verbindung der Formel 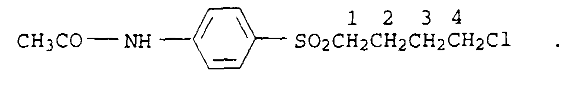
- ¹H-NMR (D₆-DMSO) :: δ = 1,5 - 1,9 (m, 4H, 2-, 3-H)
2,1 (s, 3H, CH₃)
3,3 (t, 2H, 4-H)
3,6 (t, 2H, 1-H)
7,8 (brs, 4H, Aromaten-H)
10,4 (s, 1H, NH) ppm.

### Beispiel 8

Man setzte 184 g der in Beispiel 7 beschriebenen Verbindung mit 81 g Mercaptoethanol analog Beispiel 2a) um und oxidierte anschließend analog Beispiel 2b). Man erhielt 202 g der Verbindung der Formel 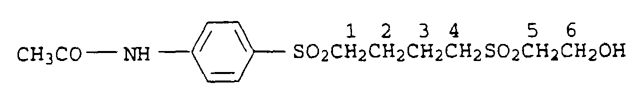
- ¹H-NMR (D₆-DMSO) :: δ = 1,6 - 1,9 (m, 4H, 2-, 3-H)
2,1 (s, 3H, CH3)
3,1 (m, 4H, 5-, 6-H)
3,3 (m, 2H, 4-H)
3,8 (t, 2H, 1-H)
7,8 (brs, 4H, Aromaten-H)
10,4 (s, 1H, NH) ppm.

### Beispiel 9

Man verfuhr analog Beispiel 1, verwendete jedoch anstelle von 4-Acetylaminobenzolsulfinsäure 227 g 4-(2-Acetylaminoethyl)benzolsulfinsäure. Man erhielt 258 g einer Verbindung der Formel 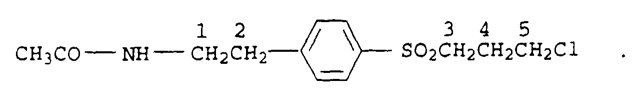
- ¹H-NMR (D₆-DMSO) :: δ = 1,8 (s, 3H, CH₃)
2,0 (q, 2H, 4-H)
2,8 (t, 2H, 2-H)
3,3 (t, 2H, 5-H)
3,4 (m, 2H, 1-H)
3,7 (t, 2H, 3-H)
7,5 (d, 2H, Aromaten-H)
7,8 (d, 2H, Aromaten-H)
8,0 (brs, 1H, NH).

### Beispiel 10

Man verfuhr analog Beispiel 2, verwendete jedoch 192 g der in Beispiel 9 beschriebenen Verbindung. Man erhielt nach dem Trocknen unter vermindertem Druck 185 g der Verbindung der Formel 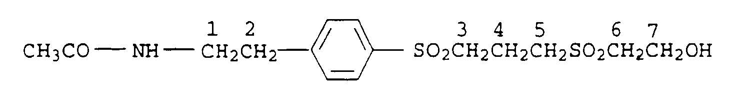
- ¹H-NMR (D₆-DMSO) :: δ = 1,8 (s, 3H, CH₃)
2,0 (m, 2H, 4-H)
2,9 , 3,2 - 3,6 (m, insgesamt 10H,
1-, 2-, 5-, 6- und 7-H)
3,8 (t, 2H, 3-H)
7,5 (d, 2H, Aromaten-H)
7,8 (d, 2H, Aromaten-H)
8,0 (brs, 1H, NH) ppm.

### Beispiel 11

50 g der in Beispiel 2b beschriebenen Verbindung wurden unter Eiskühlung bei 35 bis 40°C in 150 g 100 Gew.-%iger Schwefelsäure eingetragen und 12 h bei Raumtemperatur gerührt.

Nach vollständigem Umsatz (DC-Kontrolle) wurde auf 450 g Eis gefällt und anschließend durch Einstreuen von Soda unter Rühren und Kühlung ein pH-Wert von 5 eingestellt.

Man erhielt eine elektrolythaltige Lösung der Verbindung der Formel 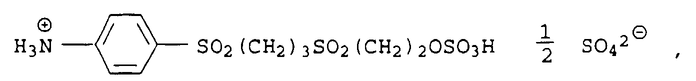 die ohne weitere Reinigung zu Folgeumsetzungen, insbesondere Farbstoffsynthesen, verwendet werden kann.

### Beispiel 12

50 g der in Beispiel 2b beschriebenen Verbindung wurden unter Eiskühlung bei 35 bis 40°C in 150 g 24 gew.-%iges Oleum eingetragen und 6 h bei 85°C nachgerührt.

Nach vollständigem Umsatz (DC-Kontrolle) wurde auf 450 g Eis gefällt, anschließend durch Einstreuen von Soda unter Rühren und Kühlung ein pH-Wert von 5 eingestellt.

Nach Abkühlen auf 0 bis 5°C wurde 1 h bei dieser Temperatur gerührt, dann wurde vom ausgefallenen Natriumsulfat abfiltriert.

Man erhielt eine elektrolythaltige Lösung der Verbindung der Formel 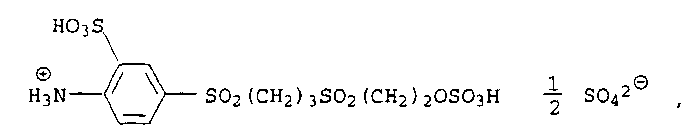 die ohne weitere Reinigung zu Folgeumsetzungen, insbesondere Farbstoffsynthesen, verwendet werden kann.

In analoger Weise werden die in der folgenden Tabelle aufgeführten Verbindungen erhalten.

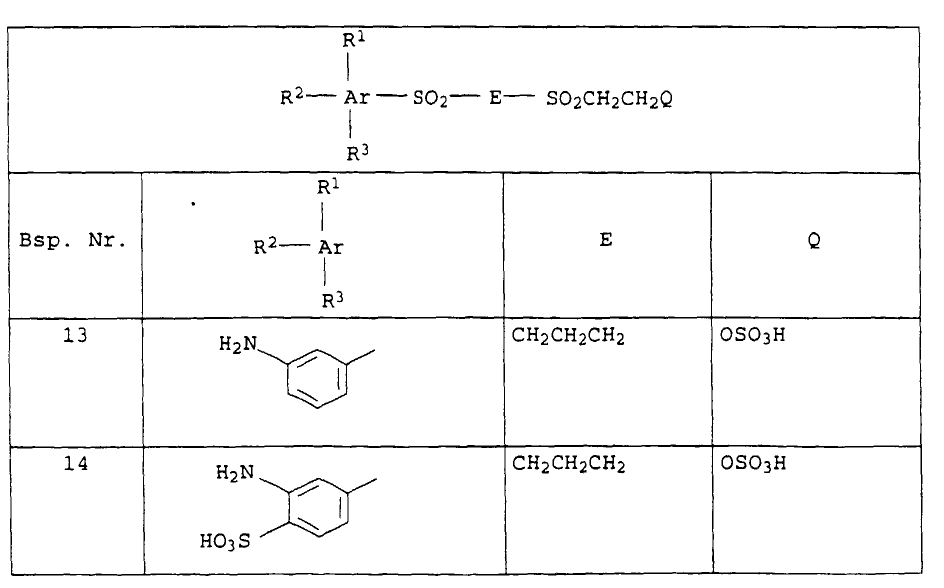

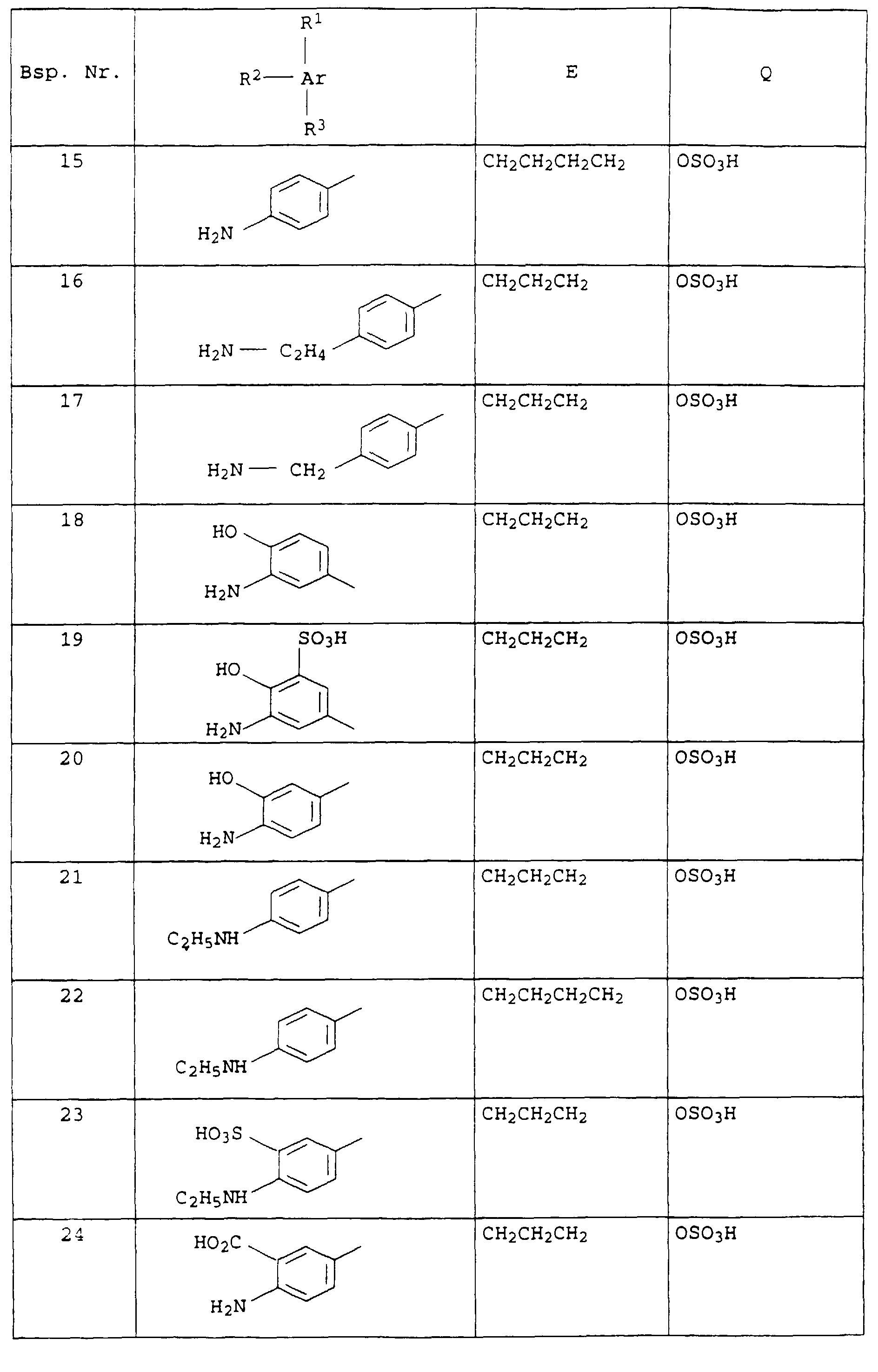

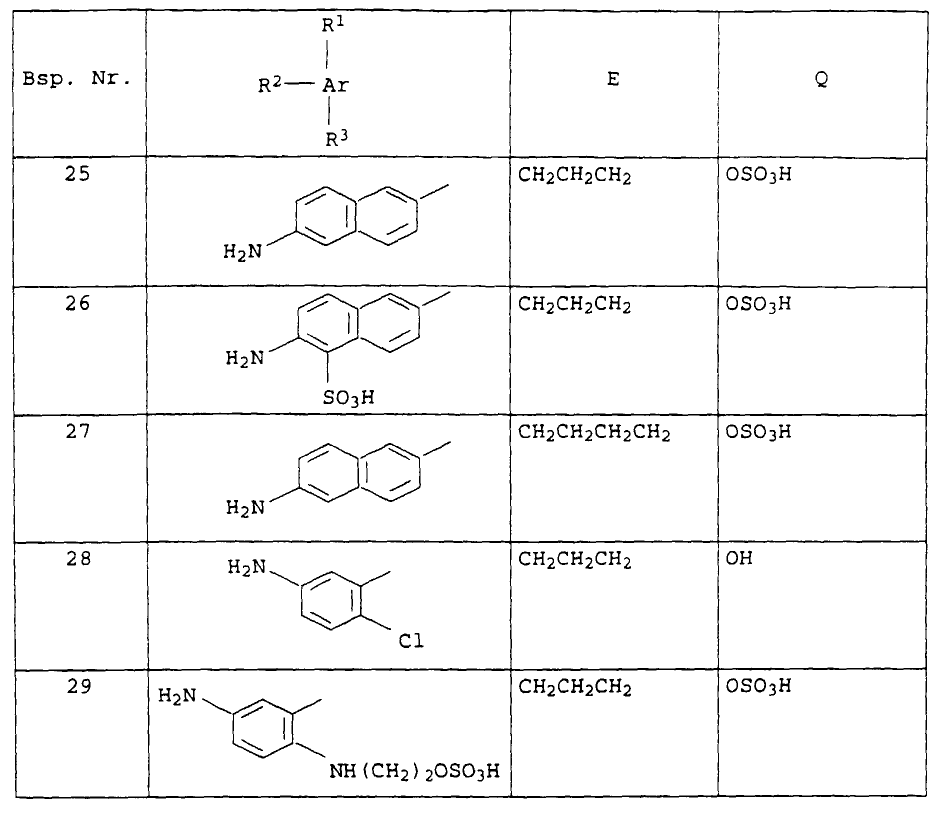

## Patentansprüche

1. Sulfonylverbindungen der Formel I 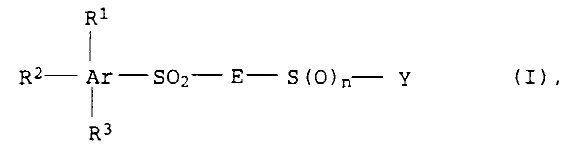 in der
n 0, 1 oder 2,
Y Vinyl oder einen Rest der Formel C₂H₄Q, worin Q für Hydroxy oder eine unter alkalischen Reaktionsbedingungen abspaltbare Gruppe steht,
E C₃-C₆-Alkylen, das durch 1 oder 2 Sauerstoffatome in Etherfunktion unterbrochen sein kann,
Ar den Rest von Benzol oder Naphthalin und
R¹, R² und R³ unabhängig voneinander jeweils Wasserstoff, C₁-C₆-Alkyl, das durch Amino oder C₁-C₄-Alkanoylamino substituiert sein kann, C₁-C₆-Alkoxy, Hydroxy, Halogen, Nitro, Amino, C₁-C₄-Alkanoylamino, Mono- oder Di-C₁-C₆-alkylamino, Hydroxysulfonyl, Carboxyl, Carbamoyl, Mono- oder Di-C₁-C₆-alkylcarbamoyl, Sulfamoyl, Mono- oder Di-C₁-C₆-alkylsulfamoyl, Cyano oder einen Rest der Formel (NH-)ₘ(CH₂-)_{q}SO₂-Y, worin m für 0 oder 1 und q für 0, 2 oder 3 stehen und Y die obengenannte Bedeutung besitzt,
bedeuten.

2. Sulfonylverbindungen nach Anspruch 1, dadurch gekennzeichnet, daß n 0 oder 2 bedeutet.

3. Sulfonylverbindungen nach Anspruch 1, dadurch gekennzeichnet, daß E C₃- oder C₄-Alkylen bedeutet.

4. Sulfonylverbindungen nach Anspruch 1, dadurch gekennzeichnet, daß Ar den Rest von Benzol bedeutet.

5. Sulfonylverbindungen nach Anspruch 1, dadurch gekennzeichnet, daß R¹ C₁-C₄-Alkyl, das durch Amino oder C₁-C₄-Alkanoylamino substituiert sein kann, Nitro, Amino oder C₁-C₄-Alkanoylamino und R² und R³ unabhängig voneinander jeweils Wasserstoff, C₁-C₄-Alkoxy, Hydroxy, Halogen, Hydroxysulfonyl oder Carboxyl bedeuten.

## Claims

1. Sulfonyl compounds of the formula I 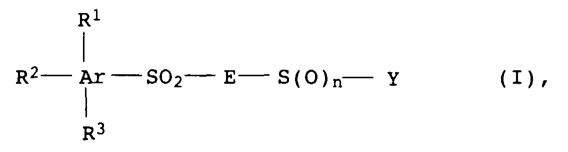 where
n is 0, 1 or 2,
Y is vinyl or a radical of the formula C₂H₄Q, where Q is hydroxyl or an alkali-detachable group,
E is C₃-C₆-alkylene with or without interruption by 1 or 2 oxygen atoms in ether function,
Ar is the radical of benzene or naphthalene, and
R¹, R² and R³ are independently of each other hydrogen, C₁-C₆-alkyl with or without amino or C₁-C₄-alkanoylamino substitution, C₁-C₆-alkoxy, hydroxyl, halogen, nitro, amino, C₁-C₄-alkanoylamino, mono- or di(C₁-C₆-alkyl)amino, hydroxysulfonyl, carboxyl, carbamoyl, mono- or di(C₁-C₆-alkyl)carbamoyl, sulfamoyl, mono- or di(C₁-C₆-alkyl)sulfamoyl, cyano or a radical of the formula (NH-)ₘ(CH₂-)_{q}SO₂-Y, where m is 0 or 1, q is 0, 2 or 3, and Y is as defined above.

2. Sulfonyl compounds as claimed in claim 1 wherein n is 0 or 2.

3. Sulfonyl compounds as claimed in claim 1 wherein E is C₃- or C₄-alkylene.

4. Sulfonyl compounds as claimed in claim 1 wherein Ar is the radical of benzene.

5. Sulfonyl compounds as claimed in claim 1 wherein R¹ is C₁-C₄-alkyl with or without amino or C₁-C₄-alkanoylamino substitution, nitro, amino or C₁-C₄-alkanoylamino and R² and R³ are independently of each other hydrogen, C₁-C₄-alkoxy, hydroxyl, halogen, hydroxysulfonyl or carboxyl.

## Revendications

1. Composés sulfonylés de formule I 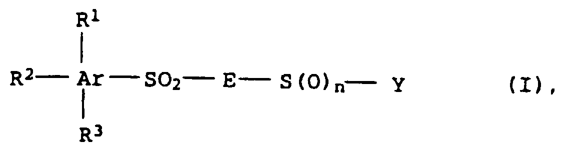 dans laquelle
n vaut 0, 1 ou 2,
Y représente un groupement vinyle, un reste de formule C₂H₄Q, où Q est mis pour un groupement hydroxy ou pour un groupement séparable dans des conditions réactionnelles alcalines,
E représente un groupement alkylène en C₃-C₆, pouvant être interrompu par 1 ou 2 atomes d'oxygène en fonction éther,
Ar représente le reste du benzène ou du naphtalène et
R¹, R² et R³ représentent chacun indépendamment les uns des autres, un atome d'hydrogène, un groupement alkyle en C₁-C₆, pouvant être substitué par un groupement amino ou alcanoylamino en C₁-C₄, un groupement alcoxy en C₁-C₆, hydroxy, un atome d'halogène, un groupement nitro, amino, alcanoylamino en C₁-C₄, mono- ou di-(alkyle en C₁-C₆)amino, hydroxysulfonyle, carboxyle, carbamoyle, mono- ou di-(alkyle en C₁-C₆)carbamoyle, sulfamoyle, mono- ou di-(alkyle en C₁-C₆)sulfamoyle, cyano ou un reste de formule (NH-)ₘ(CH₂-)_{q}SO₂-Y, où m est mis pour 0 ou 1 et q pour 0, 2 ou 3 et Y prend la signification susmentionnée.

2. Composés sulfonylés selon la revendication 1, caractérisés en ce que n vaut 0 ou 2.

3. Composés sulfonylés selon la revendication 1, caractérisés en ce que E représente un groupement alkylène en C₃ ou C₄.

4. Composés sulfonylés selon la revendication 1, caractérisés en ce que Ar représente le reste du benzène.

5. Composés sulfonylés selon la revendication 1, caractérisés en ce que R¹ représente un groupement alkyle en C₁-C₄, pouvant être substitué par un groupement amino ou alcanoylamino en C₁-C₄, un groupement nitro, amino ou alcanoylamino en C₁-C₄, et R² et R³ représentent chacun indépendamment les uns des autres un atome d'hydrogène, un groupement alcoxy en C₁-C₄, hydroxy, un atome d'halogène, un groupement hydroxysulfonyle ou carboxyle.
